# EUROPÄISCHE PATENTSCHRIFT

(11) **EP 1 115 689 B1**
(45) Veröffentlichungstag und Bekanntmachung des Hinweises auf die Patenterteilung: **18.02.2004**
(21) Anmeldenummer: 99947416.6
(22) Anmeldetag: 23.09.1999
(51) Int. Cl.: C07C 67/343, C07C 69/716

(54) **VERFAHREN ZUR HERSTELLUNG VON 6,6-DIALKOXY-5-HYDROXY-3-OXO-HEXANSÄUREESTERN**
METHOD FOR PRODUCING 6,6-DIALKOXY-5-HYDROXY-3-OXO-HEXANOIC ACID ESTERS
PROCEDE DE PRODUCTION D'ESTERS D'ACIDE 6,6-DIALCOXY-5-HYDROXY-3-OXOCAPROIQUE

(30) Priorität: 25.09.1998 EP 98118191; 03.08.1999 US 147041 P
(43) Veröffentlichungstag der Anmeldung: 18.07.2001
(73) Patentinhaber: Lonza AG, 3930 Visp (CH)
(72) Erfinder: SCHRÖER, Josef, CH-3952 Susten (CH)
(74) Vertreter: Ritthaler, Wolfgang, Dr.rer.nat.Dipl.-Chem
(86) Internationale Anmeldenummer: PCT/EP1999/007100
(87) Internationale Veröffentlichungsnummer: WO 2000/018718

(56) Entgegenhaltungen:
- WO-A-92/10461

## Beschreibung

Die Erfindung betrifft ein Verfahren zur Herstellung von 6,6-Dialkoxy-5-hydroxy-3-oxohexansäureestern der allgemeinen Formel 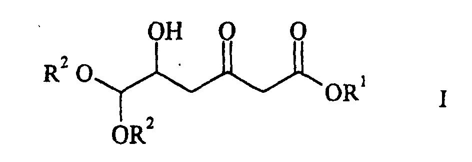 worin R¹ und R² gleich oder verschieden sind und C₁₋₆-Alkyl bedeuten.

Die genannten Verbindungen sind wichtige Synthesebausteine zur Herstellung von zahlreichen HMG-CoA-Reductase-Inhibitoren (WO-A-92/10503).

WO-A-92/10461 beschreibt ein Verfahren zur Herstellung von 3,5-Dihydroxypentansäurederivaten durch selektive Reduktion von 5-Hydroxy-3-oxopentansäurederivaten.

Gemäss WO-A-92/10503 werden diese Verbindungen durch Umsetzung von einem Essigsäureester mit einem γ-Dialkoxy-β-hydroxyester erhalten. Diese Synthese hat den Nachteil, dass letzteres Edukt schlecht zugänglich und damit teuer ist.

Die Aufgabe der Erfindung bestand darin, einen einfacheren und kostengünstigeren Zugang zu den genannten Synthesebausteinen zu entwickeln.

Die Aufgabe konnte gelöst werden mit dem Verfahren gemäss Patentanspruch 1.

Erfindungsgemäss wird darin ein Acetessigsäurealkylester der allgemeinen Formel 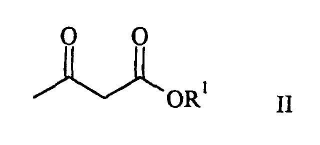 worin R¹ die genannte Bedeutung hat, in Gegenwart einer Base mit einem Aldehyd der allgemeinen Formel 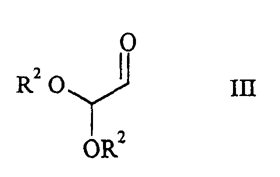 worin R² die genannte Bedeutung hat, umgesetzt.

Unter einer C₁₋₆-Alkylgruppe wird eine geradkettige oder verzweigte Alkylgruppe mit 1-6 C-Atomen verstanden, namentlich Methyl, Ethyl, 1-Propyl, n-Propyl, n-Butyl, i-Butyl, t-Butyl, Pentyl und seine Isomeren sowie Hexyl und seine Isomeren. Bevorzugt sind die namentlich aufgeführten C₁₋₄-Alkylgruppen.

Ein Vorzug des erfindungsgemässen Verfahrens besteht darin, dass die Acetessigsäurealkylester der allgemeinen Formel II grosstechnisch verfügbar sind.

Acetessigsäurealkylester der allgemeinen Formel II werden durch Umsetzung von Diketen mit dem entsprechenden Alkohol gewonnen. Der Acetessigsäurealkylester der Formel II kann auch ausgehend vom Diketen in situ zur Verfügung gestellt werden. Bevorzugte Acetessigsäurealkylester der allgemeinen Formel II sind der Methylester, der Ethylester, der n-Propylester, der i-Propylester, der n-Butylester, der i-Butylester oder der t-Butylester. Besonders bevorzugt ist der t-Butylester.

Die Aldehyde der allgemeinen Formel III sind in der Regel kommerziell erhältlich. Bevorzugter Aldehyd der Formel III ist das Glyoxal-1,1-dimethylacetal.

Zweckmässig wird das erfindungsgemässe Verfahren in einem organischen Lösungsmittel unter weitgehendem Ausschluss von Wasser durchgeführt. Geeignete Lösungsmittel sind Ether wie z.B. Tetrahydrofuran, Dioxan, Diethylether oder t-Butylmethylether, Aromaten wie Benzol oder Toluol oder Kohlenwasserstoffe wie Hexan sowie Gemische aus den genannten organischen Lösungsmitteln.

Die Umsetzungstemperatur wählt man in der Regel in einem Bereich von -80°C bis 130°C, bevorzugt im Bereich von -40°C bis 20°C.

Der Einsatz der Base hat das Ziel, das Di-Anion des Acetessigsäurealkylesters der allgemeinen Formel II zu bilden. Grundsätzlich kann diese Di-Anion-Bildung direkt mit einer starken Base, wie z.B. mit Butyllithium, Methyllithium, Phenyllithium oder Natriumamid, Lithiumdiisopropylamid oder Lithiumhexamethyldisilazan erfolgen.

Üblicherweise wird zweistufig verfahren, indem zunächst mit einer schwächeren und auch kostengünstigeren Base die -CH₂- Funktion deprotoniert wird und erst im zweiten Schritt mit der genannten starken Base die Di-Anion Bildung vollzogen wird. Als schwächere Basen eignen sich Metallhydride wie z.B. Alkali und Erdalkalihydride, vorzugsweise das Natriumhydrid, aber auch sekundäre Amine (bevorzugt Pyrrolidin), welches mit dem Acetessigsäurealkylester ein Enamin als Anionequivalent bildet. Im letzten Fall wird unter dem Di-Anion das Anion des entsprechenden Enamins verstanden.

Der gewünschte 6,6-Dialkoxy-5-hydroxy-3-oxo-hexansäureester kann auf fachmännisch übliche Weise, z.B. durch Neutralstellen des Reaktionsgemisches und anschliessende Extraktion mit einem geeigneten Lösungsmittel, erhalten werden.

### Beispiele

### Beispiel 1:

### Herstellung von 6,6-Dimethoxy-5-hydroxy-3-oxo-t-butylhexanoat

In einem 250 ml Rundkolben wurden 0,88 g (22 mmol) Natriumhydrid (55-60% in Mineralöl) in 50 ml Tetrahydrofuran bei 0°C vorgelegt. Anschliessend wurde mittels Spritze 3,16 g (20 mmol) Acetessigsäure-t-butylester während 5 - 10 min. zur Natriumhydrid-Suspension gegeben (Wasserstoffentwicklung). Es wurde 5 min. nachgerührt, dann wurde bei 0°C 13,1 ml n-BuLi-Lösung (1,6 M in Hexan) über einen Zeitraum von ca. 10 min. zum Reaktionsgemisch getropft. Man liess ca. 1 h nachrühren und tropfte anschliessend 5,7 g (22 mmol) Glyoxal-1,1-dimethlyacetal-Lösung innerhalb von 5 min. zum Reaktionsgemisch. Es wurde 3 h bei 0°C nachgerührt. Die Reaktion wurde bei 0°C mit 5 ml konzentrierter HCl gequencht (-> pH 8). Es wurde mit 30 ml Wasser verdünnt und 2 mal mit je 50 ml Diethylether extrahiert. Das durch Einengen der organischen Phasen erhaltene Rohprodukt wurde mit Säulenchromatographie gereinigt. Auf diese Weise wurde die gewünschte Verbindung in Form eines leicht viskosen, leicht gelblichen Öls isoliert. (Ausbeute: 11%)
- ¹H-NMR (400 Mhz, CDCl₃):: 1.47 (s, 9H),
2.73 (dd, 1H),
2.75 (br.s, 1H),
2.82 (dd, 1H),
3.41 (s, 2H),
3.44 (s, 3H),
3.45 (s, 3H),
4.13 (m, 1H),
4.25 (d, 1H)

### Beispiel 2:

### Herstellung von 6,6-Dimethoxy-5-hydroxy-3-oxo-t-butylhexanoat

In einem 1000 ml Doppelmantelkolben wurden 6,6 g (165 mmol) Natriumhydrid (55-60% in Mineralöl) in 400 ml Tetrahydrofuran bei 0°C vorgelegt. Anschliessend wurde mittels Tropftrichter 23,75 g (150 mmol) Acetessigsäure-t-butylester während 15 min. zur Natriumhydrid-Suspension gegeben (Wasserstoffentwicklung). Es wurde 60 min. nachgerührt, dann wurde bei 0°C 100 ml n-BuLi-Lösung (1,6 M Lösung in Hexan, 160 mmol) über einen Zeitraum von ca. 40 min. zum Reaktionsgemisch getropft. Man liess ca. 25 min nachrühren und tropfte anschliessend 34,7 g (150 mmol) Glyoxal-1,1-dimethylacetal (45%ige Lösung in TBME) innerhalb von 30 min. zum Reaktionsgemisch. Es wurde 20 min. bei 0°C und 1h bei 20°C nachgerührt. Die Reaktion wurde bei 0°C mit 37 ml konzentrierter HCl und 250 ml Wasser gequencht (-> pH 8). Es wurde 2 mal mit je 250 ml Diethylether extrahiert. Das durch Einengen der organischen Phasen erhaltene Rohprodukt wurde mit Säulenchromatographie gereinigt. Auf diese Weise wurde die gewünschte Verbindung in reiner Form isoliert.
Ausbeute: 17,3 g, 44%

### Beispiel 3:

### Herstellung von 3-N-Pyrrolidino-t-butyl-but-2-enoat

Eine Lösung von Acetessigsäure-t-butylester (7,91 g, 50 mmol) in 50 ml Toluol wird mit 3,75 g Pyrrolidin (52,5 mmol) versetzt und 1,5 h am Wasserabscheider refluxiert. Nach dieser Zeit hat sich die theoretische Menge Wasser abgeschieden. Das Enamin kann in praktisch quantitativer Ausbeute durch Einengen des Reaktionsgemisches gewonnen werden.
- ¹H-NMR (400 MHz, CDCl₃):: 1.46 (s, 9H),
1.91 (m, 4H),
2.43 (s, 3H),
3.27 (br.s, 4H),
4.41 (s, 1H)

## Patentansprüche

1. Verfahren zur Herstellung von 6,6-Dialkoxy-5-hydroxy-3-oxo-hexansäureestern der allgemeinen Formel: 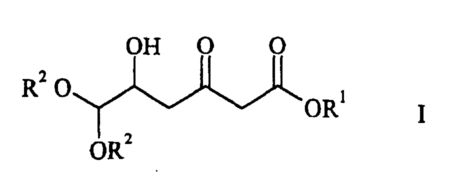 worin R¹ und R² gleich oder verschieden sind und C₁₋₆-Alkyl bedeuten, **dadurch gekennzeichnet, dass** ein Acetessigsäurealkylester der allgemeinen Formel 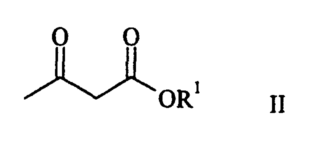 worin R¹ die genannte Bedeutung hat, in Gegenwart einer Base mit einem Aldehyd der allgemeinen Formel 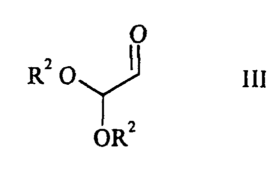 worin R² die genannte Bedeutung hat, umgesetzt wird.

2. Verfahren nach Anspruch 1, **dadurch gekennzeichnet, dass** die Umsetzung in einem organischen Lösungsmittel unter weitgehendem Ausschluss von Wasser durchgeführt wird.

3. Verfahren nach Anspruch 1 oder 2, **dadurch gekennzeichnet, dass** die Umsetzung bei einer Temperatur von -80°C bis 130°C durchgeführt wird.

4. Verfahren nach einem der Ansprüche 1 bis 3, **dadurch gekennzeichnet, dass** eine Base verwendet wird, die in der Lage ist, das Di-Anion des Acetessigsäurealkylesters der allgemeinen Formel II zu bilden.

5. Verfahren nach Anspruch 4, **dadurch gekennzeichnet, dass** zunächst mit einer schwächeren Base eine Deprotonierung an der -CH₂- Funktion des Acetessigsäurealkylesters der allgemeinen Formel II erreicht wird bzw. durch Enaminbildung ein entsprechendes Anionequivalent gebildet wird und erst anschliessend mit einer stärkeren Base das Di-Anion gebildet wird.

## Claims

1. Process for preparing 6,6-dialkoxy-5-hydroxy-3-oxohexanoic acid esters of the general formula 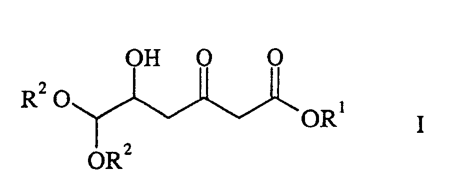 in which R¹ and R² are identical or different and are C₁₋₆-alkyl, **characterized in that** an alkyl acetoacetate of the general formula 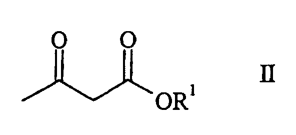 in which R¹ is as defined above, is reacted in the presence of a base with an aldehyde of the general formula 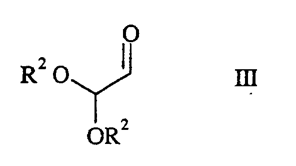 in which R² is as defined above.

2. Process according to Claim 1, **characterized in that** the reaction is carried out in an organic solvent in the substantial absence of water.

3. Process according to Claim 1 or 2, **characterized in that** the reaction is carried out at a temperature of from -80°C to 130°C.

4. Process according to any one of Claims 1 to 3, **characterized in that** a base is used which is capable of forming the dianion of the alkyl acetoacetate of the general formula II.

5. Process according to Claim 4, **characterized in that** using a relatively weak base at first a deprotonation at the -CH₂- function of the alkyl acetoacetate of the general formula II is effected or a corresponding anion equivalent is formed by enamine formation, respectively, and only afterwards the dianion is formed using a relatively strong base.

## Revendications

1. Procédé de préparation d'esters d'acides 6,6-dialcoxy-5-hydroxy-3-oxo-hexanoïques de formule générale : 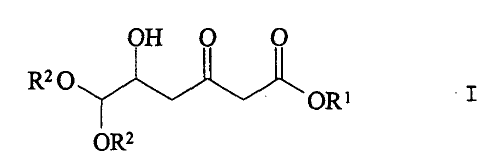 dans laquelle R¹ et R² sont identiques ou différents et représentent un alkyle en C₁ à C₆, **caractérisé en ce que** l'on fait réagir un ester alkylique d'acide acétoacétique de formule générale : 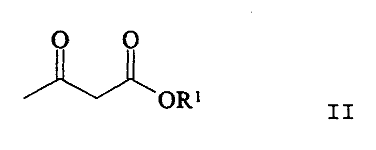 dans laquelle R¹ a la signification indiquée, en présence d'une base avec un aldéhyde de formule générale : 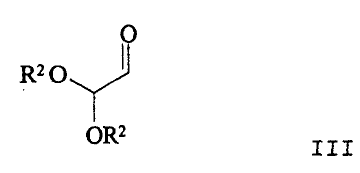 dans laquelle R² a la signification indiquée.

2. Procédé selon la revendication 1, **caractérisé en ce que** la réaction se fait dans un solvant organique avec une large élimination de l'eau.

3. Procédé selon la revendication 1 ou la revendication 2, **caractérisé en ce que** la réaction se fait à une température de -80°C à 130°C.

4. Procédé selon l'une des revendications 1 à 3, **caractérisé en ce que** l'on utilise une base qui est capable de former le dianion de l'ester alkylique d'acide acétoacétique de formule générale II.

5. Procédé selon la revendication 4, **caractérisé en ce que** l'on effectue d'abord, au moyen d'une base plus faible, une déprotonation au niveau de la fonction -CH₂- de l'ester alkylique d'acide acétoacétique de formule générale II ou bien **en ce que**, par formation d'énamine, on forme un équivalent anion correspondant et ensuite seulement on forme le dianion au moyen d'une base plus forte.
